# EUROPEAN PATENT SPECIFICATION

(11) **EP 2 875 871 B1**
(45) Date of publication and mention of the grant of the patent: **21.02.2018**
(21) Application number: 12881216.1
(22) Date of filing: 20.09.2012
(51) Int. Cl.: B05B 7/16, B05B 1/28, A61L 9/14, B05B 7/00, B05B 7/24

(54) **AIR FRESHENER ATOMISER AND METHOD**
LUFTERFRISCHERZERSTÄUBER UND VERFAHREN
NÉBULISEUR ASSAINISSEUR D'AIR ET PROCÉDÉ

(30) Priority: 20.07.2012 ES 201200743
(43) Date of publication of application: 27.05.2015
(73) Proprietor: Europea de Servicios e Higiene Euro-Servhi S.A., 48002 Bilbao, Vizcaya (ES)
(72) Inventor: VILLAR CALVO, Alvaro, 48002 Bilbao, Vizcaya (ES)
(74) Representative: Juncosa Miro, Jaime
(86) International application number: PCT/ES2012/000244
(87) International publication number: WO 2014/013091

(56) References cited:
- EP-A1- 0 608 176
- EP-A1- 1 449 502
- WO-A1-91/00479
- FR-A1- 2 780 664
- FR-A1- 2 964 887
- US-A- 5 461 695
- US-A1- 2004 046 053
- US-A1- 2006 145 368

## Description

### Field of the Art

The present invention relates to an atomizer which is related to fragrance dispensing air freshener apparatus used for conditioning the environment of closed areas both in homes and in business premises in an automatic manner and depending on the needs of each case, with variants in the size of the area and of the environment. Furthermore, the present invention also relates to a novel process whereby achieving a method for combining a load with air to produce a wet gasification product and finally change it into an atomized dry gasification product so that it acts as an air freshener, without any risk of fire or of seeing the atomized load.

### Background of the Invention

There are various air freshener apparatus and devices which are widely known given that they are usually promoted with advertising campaigns when they are for use at home, although atomizers for use at home are few in number, rather they are more for use at business premises, although devices acting and used as dispensing air fresheners are very well known.

On the other hand, there are also dispensers the purpose of which is not so much to freshen the air or remove bad smells from the environment, but to simply maintain the humidity of the environment, although said air freshener and humidifying functions are usually combined.

The present invention consists of an apparatus which, even though it initially produces a first atomization in a chamber by combining pressurized air with the liquid load of the air freshener, finally results in a gasification product that can be considered dry exiting into the environment, which dry product has advantages with respect to that already known given that it is not flammable and cannot be seen.

Based on the technological patent research that has been conducted, various documents resorting to atomizing and vaporizing products in order to apply or transfer same to different means have been found. Capturing and collecting larger droplets or particles are relatively important in all the documents found, particularly for achieving more homogeneous and efficient product application. Said capturing is achieved by means of changing the trajectory or reducing the speed of the atomized flow. In this regard, reference can be made to patent document US5461695; however, the device of this document does not incorporate the atomizing chambers referred to in the device of the present invention. Although patent document WO9100479 has deflecting means to divert larger droplets, heating is performed after atomization so it is not consistent with the technical features described in the present invention. In contrast, patent document EP1449502 discloses heating the air before atomization and removing droplets from the atomized flow; however, the means used for retaining atomized droplets are different from those applied in the present invention, and the atomized liquid is demineralized water and not a water-alcohol solution. Finally, patent document FR2780664 discloses a device which also preheats the air with which the atomized product is mixed, but the atomized product outlet and its outlet chamber are not at all comparable with the present invention. An air freshener atomizer according to the preamble of claim 1 is known from EP 0 608 176 A1. In short, the technical features referred to in the present invention are in no way anticipated by the known state of the art.

### Description of the Invention

This air freshener atomizer consists of a heated aeration pump or heating compressor which is connected to an injector unit arranged in a specific and characteristic manner for combining two channels, according to the object of the present invention, which injects the air heated by the heating compressor together with the air freshener liquid, having a special and characteristic formulation, all this at a specific compensated pressure and with the Venturi effect, giving rise to gasification of hot wet air with the compounds of the injected air freshener liquid.

Said injector unit further comprises a series of atomizing chambers which, as a result of the design thereof in compartments of decreasing volume, converts the wet gasification product into a dry gasified product, and therefore with the advantages of resulting in a dosage exiting as a non-flammable and visually non-perceptible product.

Said atomized product is dispensed into the environment through an atomizing nozzle arranged at the outlet of the second atomizing chamber.

All the elements are integrated and combined within a frame with all its elements custom-designed and custom-arranged to form it in a compact and integrated manner in an assembly giving rise to an air freshener atomizer, the object of the invention, along with a method, starting by combining pressurized hot air with an air freshener liquid, a water-alcohol solution, forming a wet gasified product in the first atomizing chamber (tapered conical chamber), which gasified product then goes into the second atomizing chamber (endless vertical chamber) forming a dry gasified product which exits same, while the possible wet particles return by means of gravitational effect through a return conduit to the start of the combined dual-channel injection (combined injector).

The invention relates to previously heating air, whereby atomization of a potentially flammable water-alcohol solution will be performed by means of the Venturi effect, using two chambers that are in successive phases, with morphological shapes of the walls thereof and the tapered shape thereof allowing vaporization optimization, achieving an atomized product that can be considered dry and collecting droplets of the non-vaporized product which return to the main container.

### Brief Description of the Drawings

The different parts and arrangements of the improved air freshener atomizer of the present invention are described below by means of drawings, which parts and arrangements are designed and arranged in a specific manner that is necessary to give rise to the present invention.

The foregoing and other features and advantages will be more clearly understood based on the following detailed description of an embodiment in reference to the attached drawing in which:

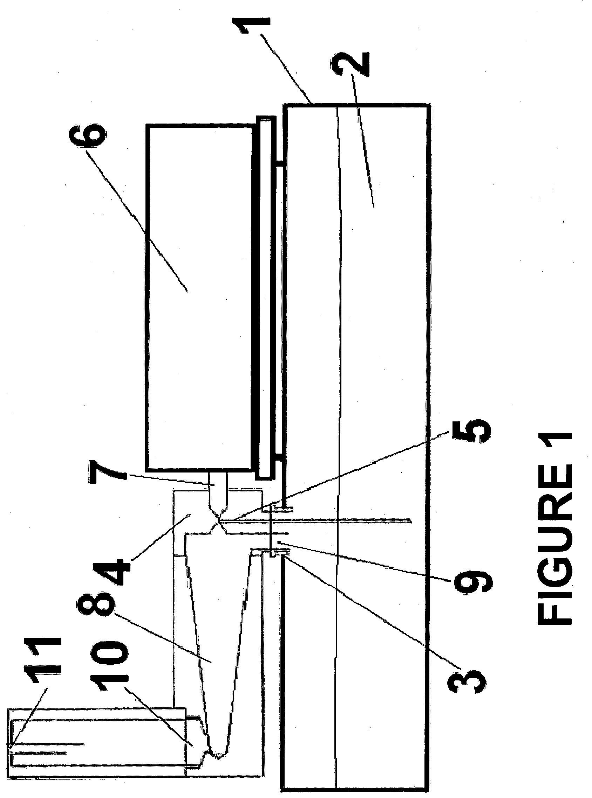

Figure 1 shows the air freshener atomizer object of the invention, in which case it has a container (1), for a load (2), having arranged in its upper part in the mouth (3) a combined dual-channel injector unit (4) having an injection tube (5); in addition the combined dual-channel injector unit (4) is connected to the heating compressor (6) through the inlet tube (7) thereof, and the injector unit (4) in turn having a first atomizing chamber (tapered conical chamber) (8), having at the lower zone of its widest end a return conduit (9) returning to the container (1), and having attached to the upper zone of its narrowest end a second atomizing chamber (endless vertical chamber) (10), having in its upper region the atomizing nozzle (11), which is the outlet.

### Detailed Description of an Embodiment

In view of what has been described above, the present invention relates to an air freshener atomizer which, on one hand, consists of the arrangement of specific elements and devices placed in a specific manner and with a very specific design, in addition to consisting of a new method of dry gasification for freshening air through an air freshener atomizer, and it therefore has:
A container (1) which contains the load (2) and which has in its upper region the load mouth (3), in which there is placed an injector unit (4) having arranged therein an injection tube (5) which is introduced on one side in the container (1) for contacting the load (2), which will be atomized when combining it with the heated pressurized air supplied thereto by the heating compressor (6) through the tube (7) to the injector unit (4), resulting in a load (2) which, when combined with the pressurized air, is converted into a wet gasified load that goes into the atomizing chamber (tapered conical chamber) (8) which is designed with a decreasing internal volume distribution thereof and which has in the part thereof with greater volume a return conduit (9) for returning to the container (1), through which part of the wet components of the wet gasified load remaining from the conversion phases occurring in the injector unit returns due to the shape of said atomizing chamber (tapered conical chamber) (8) and which re-enters the container (1) and is mixed with the load (2) again. At the other end, which is narrower, of the atomizing chamber (tapered conical chamber) (8), there is attached in its upper region a second atomizing chamber (endless vertical chamber) (10) which receives the semi-dry gasified load and converts it into dry gasified load, and the atomized dry gasified load exits the atomizer through an atomizing nozzle (11) at the outlet to freshen air.

The invention therefore relates to an air freshener atomizer carrying out a new method whereby functional operation of a combined dual-channel injector unit is performed by: a) introducing in the injector unit (4) through channel 1 of the tube (7) air from the environment through the heating compressor (6), said heating compressor also preheats the air as a result of it having a special leak-tight design and an has an electromagnet acquiring temperature during operation thereof, where the special internal design of the compressor makes the aspired ambient air goes through said electromagnet heating same before being introduced in the drive membranes and discharged through channel 1 of the tube (7); b) introducing through channel 2 of the injection tube (5) a water-alcohol solution having a specific and special formulation, different from the water-alcohol solutions used in spray air freshener systems. The solution is introduced into the injector through an intake valve designed for such purpose which is only activated when the heating compressor is in the ON position and is integrated in the injector (4) assembly.

A wet gasification process occurs with the co-injection of the components from channels 1 and 2. The design of both injectors in a tapered conical shape makes the material introduced through same experiences overpressure, which generates a change in the molecular structures thereof, combining both injectors in a common outlet which allows wet gasification of the injected elements. The overpressure in the air generates a higher hydrogen and oxygen index therein, which with the contribution of the air freshening water-alcohol solution, gives rise to the resulting intermediate product of the process.

Finally, dry gasification occurs in which the wet gasification product is converted into a dry gasification product. The common outlet of the injectors is connected in parallel with the atomizing chambers (8) and (10), conversion of the wet gasification product into a dry gasification product taking place in said chambers. At this point of the process, it must be pointed out that since the injected product is wet and has a water-alcohol solution, it is flammable. The solution resulting in the common outlet of the injectors is driven into the atomizing chamber (tapered conical chamber) (8), the decreasing internal volume distribution thereof making the molecular structure of the injected solution breaks down again, giving rise on one hand to the so-called semi-dry gasification product, and on the other hand the remaining wet particles that return, being dried again and dissipated by direct contact with the air being driven in, and other non-dissipated particles return through the return conduit (9) to the container (1), to be re-injected with the load (2). The second atomizing chamber (endless vertical chamber) (1) receives the semi-dry gasified load and converts it into a dry gasified product. Finally, the dry gasification product goes through the atomizing nozzle (11) to exit and produce its air freshening effects, where said dry atomization product resulting from a combination of air and water-alcohol product is not flammable, giving rise to the new production of a novel dry atomized product through the arrangement and method object of the present invention.

## Claims

1. - An air freshener atomizer, comprising:
- an injector unit (4);
- a compressor (6) driving air into said injector unit (4) through an air injection tube (7) ;
- a container (1) containing a load (2) which is a solution to be dispensed,
- a first atomizing chamber (8) having an input end and an output end; and
- a second atomizing chamber (10) having an input end in fluidic communication with said output end of the first atomizing chamber (8) and an output end having an atomizing nozzle (11);
- a liquid injection tube (5) arranged in the injector unit (4) in fluidic communication with said load (2);
such that the load (2) is combined with said air driven by the compressor (6) by means of the Venturi effect and atomized in the injector unit (4), the atomized load (2) goes into the first atomizing chamber (8) through the input end thereof, passes there through, then goes into the second atomizing chamber (10) through the input end thereof, and then the atomized load (2) exits the air freshener atomizer through said atomizing nozzle (11);
**characterized in that**:
- said compressor (6) is a heating compressor (6) driving heated pressurized air into the injector unit (4) through said air injection tube (7);
- said first atomizing chamber (8) is a tapered conical first atomizing chamber (8) having a decreasing internal volume distribution from said input end to said output end, and is arranged in a horizontal position in alignment with the air injection tube (7);
- said second atomizing chamber (10) is a vertical second atomizing chamber (10) fluidically communicating with an upper zone of a narrowest end of the tapered conical first atomizing chamber (8) and having at an upper region thereof the atomizing nozzle (11); and
- a return conduit (9) is provided fluidically communicating a lower zone of a widest end of the first atomizing chamber (8) with said container (1) for returning remaining wet particles to the container (1);
whereby the atomized load (2) is a potentially flammable water alcohol solution, which is converted into a semi-dry gasified load in the first atomizing chamber (8) and said semi-dry gasified load is received and converted into a non-flammable dry gasified product in the vertical second atomizing chamber (10).

2. The air freshener atomizer according to claim 1, wherein the heating compressor (6) has drive membranes which drive ambient air towards the air injection tube (7) and an electromagnet which heats the ambient air before being introduced to said drive membranes.

## Patentansprüche

1. Lufterfrischerzerstäuber, umfassend:
- eine Einspritzereinheit (4);
- einen Kompressor (6), welcher Luft in die genannte Einspritzereinheit (4) durch ein Lufteinspritzrohr (7) antreibt;
- einen Behälter (1) enthaltend eine Ladung (2), welche eine zu dispensierende Lösung ist,
- eine erste Zerstäubungskammer (8), welche ein Eingangsende und ein Ausgangsende aufweist; und
- eine zweite Zerstäubungskammer (10), welche ein Eingangsende in Fluidverbindung mit dem genannten Ausgangsende der ersten Zerstäubungskammer (8) und ein Ausgangsende mit einer Zerstäuberdüse (11) aufweist;
- ein Flüssigkeitseinspritzrohr (5), welches in der Einspritzereinheit (4) in Fluidverbindung mit der genannten Ladung (2) angeordnet ist;
so dass die Ladung (2) mit der genannten, vom Kompressor (6) mittels des Venturi-Effekts angetriebenen Luft kombiniert wird und in der Einspritzereinheit (4) zerstäubt wird, die zerstäubte Ladung (2) in die erste Zerstäubungskammer (8) durch das Eingangsende derselben hineingeht, durch dieselbe hindurchgeht, dann in die zweite Zerstäubungskammer (10) durch das Eingangsende derselben hineingeht, und dann tritt die zerstäubte Ladung (2) aus dem Lufterfrischerzerstäuber durch die genannte Zerstäuberdüse (11) aus;
**dadurch gekennzeichnet, dass**:
- der genannte Kompressor (6) ein Heizkompressor (6) ist, welcher geheizte Druckluft in die Einspritzereinheit (4) durch das genannte Lufteinspritzrohr (7) antreibt;
- die genannte erste Zerstäubungskammer (8) eine erste konisch zulaufende Zerstäubungskammer (8) mit einer verringerten Innenvolumenverteilung vom genannten Eingangsende zum genannten Ausgangsende ist, und welche in einer horizontalen Stellung mit dem Lufteinspritzrohr (7) fluchtend angeordnet ist;
- die genannte zweite Zerstäubungskammer (10) eine zweite vertikale Zerstäubungskammer (10) in Fluidverbindung mit einer oberen Zone eines engsten Endes der ersten konisch zulaufenden Zerstäubungskammer (8) ist und welche an einem oberen Bereich derselben die Zerstäuberdüse (11) aufweist; und
- eine Rückführleitung (9) vorgesehen ist, welche eine Fluidverbindung zwischen einer unteren Zone eines weitesten Endes der ersten Zerstäubungskammer (8) und dem genannten Behälter (1) herstellt, um die restlichen feuchten Partikel zum Behälter (1) zurückzuführen;
wodurch die zerstäubte Ladung (2) eine potentiell entzündliche Wasser-AlkoholLösung ist, welche in der ersten Zerstäubungskammer (8) in eine halbtrocken vergaste Ladung umgewandelt wird, und die genannte halbtrockene vergaste Ladung wird in der zweiten vertikalen Zerstäubungskammer (10) empfangen und darin in ein nichtenzündbares trockenes vergastes Produkt umgewandelt.

2. Lufterfrischerzerstäuber nach Anspruch 1, wobei der Heizkompressor (6) Treibmembrane, welche Umgebungsluft in Richtung des Lufteinspritzrohrs (7) antreiben, und einen Elektromagnet, welcher die Umgebungsluft erhitzt bevor sie in die genannten Treibmembrane eingeführt wird, aufweist.

## Revendications

1. Un atomiseur de désodorisant comportant :
- Une unité d'injection (4) ;
- un compresseur (6) entraînant de l'air à cette unité d'injection (4) à travers un tuyau d'injection (7),
- un récipient (1) contenant une charge (2) qui est une solution à distribuer,
- une première chambre d'atomisation (8) ayant une extrémité d'entrée et une extrémité de sortie ; et
- une deuxième chambre d'atomisation (10) ayant une extrémité d'entrée en communication fluidique avec cette extrémité de sortie de la première chambre d'atomisation (8) et une extrémité de sortie ayant une buse d'atomisation (11) ;
- un tuyau d'injection de liquide (5) aménagé dans l'unité d'injection (4) en communication fluidique avec cette charge (2) ;
de sorte que la charge (2) est combinée avec cet air entraîné par le compresseur (6) au moyen de l'effet Venturi et elle est atomisé dans l'unité d'injection (4), la charge atomisée (2) allant à la première chambre d'atomisation (8) à travers son extrémité d'entrée, elle y passe à travers, puis va à la deuxième chambre d'atomisation (10) à travers son extrémité d'entrée et ensuite la charge atomisée (2) fait sortir le désodorisant de l'atomiseur à travers cette buse d'atomisation (11) ;
**Caractérisée en ce que** :
- ce compresseur (6) est un compresseur chauffant (6) entraînant de l'air sous pression chauffé à l'unité d'injection (4) à travers ce tuyau d'injection d'air (7) ;
- cette première chambre d'atomisation (8) est une première chambre d'atomisation fuselée conique (8) ayant une distribution de volume interne décroissant depuis cette extrémité d'entrée à cette extrémité de sortie et elle est aménagée en position horizontal d'alignement avec le tuyau d'injection d'air (7) ;
- cette deuxième chambre d'atomisation (10) est une deuxième chambre d'atomisation verticale (10) en communication fluidique avec une région supérieure d'une extrémité plus étroite de la première chambre d'atomisation fuselée conique (8) et ayant à sa région supérieure la buse d'atomisation (11) ; et
- un conduit de retour (9) est prévu en communication fluidique avec une région inférieure d'une extrémité plus large de la première chambre d'atomisation (8) avec ce récipient (1) pour retourner les particules humides restantes au récipient (1) ;
où la charge atomisée (2) est une solution eau-alcool potentiellement inflammable, qui est convertie en une charge gazéifiée demi-sèche dans la première chambre d'atomisation (8) et cette charge gazéifiée demi-sèche est reçue et convertie en un produit gazéifié sec non-inflammable dans la deuxième chambre d'atomisation verticale (10).

2. L'atomiseur de désodorisant conformément à la revendication 1, où le compresseur chauffant (6) possède des membranes d'entraînement qui entraînent l'air ambiant vers le tuyau d'injection d'air (7) et un électro-aimant qui chauffe l'air ambiant avant d'être introduit à ces membranes.
